# EUROPÄISCHE PATENTSCHRIFT

(11) **EP 2 337 545 B1**
(45) Veröffentlichungstag und Bekanntmachung des Hinweises auf die Patenterteilung: **15.08.2012**
(21) Anmeldenummer: 09778483.9
(22) Anmeldetag: 11.09.2009
(51) Int. Cl.: A61K 8/06, A61K 8/35, A61K 8/40, A61K 8/49, A61K 8/97, A61Q 17/04, A61K 8/34

(54) **UV-FILTER HALTIGE O/W-WIRKSTOFFEMULSION**
OIL/WATER ACTIVE INGREDIENT EMULSION CONTAINING UV FILTER
ÉMULSION DE SUBSTANCE ACTIVE HUILE DANS EAU CONTENANT UN FILTRE UV

(30) Priorität: 16.09.2008 DE 102008048328
(43) Veröffentlichungstag der Anmeldung: 29.06.2011
(60) Teilanmeldung: 11008456.3
(73) Patentinhaber: Beiersdorf AG, 20253 Hamburg (DE)
(72) Erfinder: RUPPERT, Stephan, 20259 Hamburg (DE); STEIKERT, Claudia, 22529 Hamburg (DE); BLOHM, Alexandra, 20257 Hamburg (DE)
(86) Internationale Anmeldenummer: PCT/EP2009/006608
(87) Internationale Veröffentlichungsnummer: WO 2010/031522

(56) Entgegenhaltungen:
- EP-A1- 1 566 172
- DE-A1- 10 356 164
- DE-A1- 10 356 187
- NEUES AUS DERMATOLOGIE, KOSMETIK UND WUNDVERSORGUNG - BEITRÄGE ZUM STAND DER TECHNIK, Nr. 18, Oktober 2007 (2007-10), XP040423227
- DATABASE WPI Section Ch, Week 200478 Thomson Scientific, London, GB; Class B04, AN 2004-785480 XP002563281 "External use composition containing oil-soluble licorice extract and its stabilization method" & CN 1 520 811 A (MARUZEN PHARMA CO LTD) 18. August 2004 (2004-08-18)
- CHEMICAL ABSTRACTS, Bd. 54, Nr. 2, 16. November 1998 (1998-11-16), Columbus, Ohio, US; abstract no.: 142:43426, CHO, HWAN ILL ET AL.: "Liquid crystal gel for stabilized oil soluble extracts of glycyrrhizae radix and cosmetic compositions" XP002563280 & KR 0 154 366 B1 (LG CHEMICAL LTD [KR]) 16. November 1998 (1998-11-16)
- TARA E. GOTTSCHALCK ET AL.: "International Cosmetic Ingredient, Dictionary and Handbook, 11th edition, volume 1" 2006, THE COSMETIC, TOILETRY, AND FRAGRANCE ASSOCIATION , XP002563312 Eintrag "BIS-ETHYLHEXYLOXYPHENOL METHOXYPHENYL TRIAZINE" Seite 252
- MICHAEL CASWELL: "The Chemistry and Manufacture of Cosmetics, Volume II - Formulating, 3rd Edition, Chapter 4: Sunscreen Formulations and Tanning Formulations" 2000, ALLURED PUBLISHING CORPORATION , XP009128340 Formula 1 auf S. 87

## Beschreibung

Die vorliegende Erfindung betrifft eine kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt bzw. Licochalkon A,
d) Ethanol
sowie deren Herstellung und Verwendung.

Die Haut ist das größte Organ des Menschen. Sie hat eine Vielzahl lebenswichtiger Funktionen zu erfüllen, beispielsweise die Wärmeregulation und die Barrierefunktion gegen das Austrocknen der Haut und des gesamten Organismus sowie als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Haut ist einer Vielzahl an physikalischen, chemischen und biologischen Belastungen ausgesetzt. Eine Vielzahl dieser Belastungen führt aus unterschiedlichen Gründen zu einer vorübergehenden oder dauerhaften Rötung der Haut.

Damit die Haut ihre biologischen Funktionen erfüllen kann, bedarf sie der regelmäßigen Reinigung und Pflege. Um die Regeneration der Haut zu fördern, sie vor einer vorzeitigen Alterung zu schützen oder Reizungen zu vermeiden werden kosmetischen Hautpflegeprodukten in der Regel Wirkstoffe zugesetzt.

Zu den in der Hautpflege eingesetzten Wirkstoffen gehören beispielsweise Süßholzextrakte. Der Schlüsselwirkstoff von Süßholzextrakten, insbesondere von *Glycyrrhiza inflata*, ist dabei die Verbindung Licochalcon A, welche die folgende Struktur aufweist:

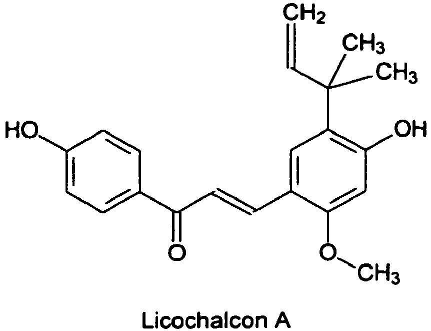

Licochalcon A enthaltende Süßholzextrakte werden dabei insbesondere gegen Hautreizungen wie Rötungen eingesetzt.

Nachteilig am Stande der Technik von Süßholzextrakten enthaltenden kosmetischen Zubereitungen (insbesondere, wenn diese Extrakte Licochalcon A enthalten), ist jedoch der Umstand, dass die Extrakte und insbesondere das Licochalcon A nicht besonders lagerstabil sind. Werden diese Zubereitungen über einen längeren Zeitraum und insbesondere bei höheren Temperaturen gelagert, kommt es zu einem Abbau des/der Wirkstoffe in der Zubereitung und das Kosmetikum verliert langsam an Wirksamkeit. Dieser Wirksamkeitsverlust tritt besonders stark bei O/W-Emulsionen (Öl-in-Wasser-Emulsionen) auf.

Es war daher die Aufgabe der vorliegenden Erfindung, die Nachteile des Standes der Technik zu beseitigen und lagerstabile bzw. temperaturstabile kosmetische Zubereitungen zu entwickeln, die Süßholzextrakte bzw. Licochalcon A enthalten.

Darüber hinaus war es die Aufgabe der vorliegenden Erfindung, eine Zubereitung zu entwickeln, die sich einfach und kostengünstig herstellen lässt.

Überraschend gelöst wird die Aufgabe durch eine kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt und
d) Ethanol.

Die Aufgabe wird auch überraschend gelöst durch eine kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Licochalcon A und
d) Ethanol.

Überraschend gelöst wird die Aufgabe insbesondere durch die Verwendung einer UV-Filterkombination aus
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan), zur Erhöhung der Lagerstabilität von Licochalkon A bzw. Süßholzextrakten in kosmetischen O/W-Emulsionen.

Erfindungsgemäß ist dabei insbesondere das Verfahren zur Herstellung einer Licochalkon A und/oder Süßholzextrakt enthaltenden O/W-Emulsion, dadurch gekennzeichnet, dass
a) die heiße Wasserphase vorgelegt wird,
b) in die heiße Wasserphase die erhitzte Ölphase, enthaltend 2-Ethylhexyl-2-cyano-3,3-di-phenylacrylat, 4-(tert.-Butyl)-4'-methoxydibenzoylmethan, und
d) 2,4-Bis-{[4-(2-ethyl-hexyloxy)-2-hydroxy]-phenyl}-6-(4-methoxyphenyl)-1,3,5-triazin (INCI: Aniso Triazin) und ggf. weitere öllösliche UV-Filter wie 2,4-Bis-{[4-(2-ethyl-hexyloxy)-2-hydroxy]-phenyl}-6-(4-methoxyphenyl)-1,3,5-triazin, hinzugefügt wird,
c) anschließend beide Phasen zu einer O/W-Emulsion homogenisiert werden,
d) die Zubereitung abgekühlt wird,
e) der in Ethanol gelöste Süßholzextrakt bzw. das in Ethanol gelöste Licochalkon A in die Emulsion eingerührt wird und anschließend die Parfümstoffe und Konservierungsmittel zugesetzt werden, sowie
eine kosmetische O/W-Emulsion hergestellt nach diesem Verfahren.

Zwar kennt der Fachmann an sich kosmetische Zubereitungen mit Süßholzextrakten und insbesondere mit Licochalcon A. So beschreiben die deutschen Offenlegungsschriften DE 102 24 387.5, DE 103 52 368.5, DE 103 52 367.7, DE 103 56 723.2, DE 103 56 175.7, DE 103 42 212.9, DE 103 52 369.3, DE 103 56 187.0. DE 103 57 451.4. DE 103 57 452.2, DE 103 56 164.1, DE 103 56 870.0, DE 103 56 869.7 und DE 103 56 866.2 Licochalcon A enthaltende Zubereitungen. Diese Schriften konnten jedoch nicht den Weg zur vorliegenden Erfindung weisen, da in den offenbarten Rezepturen entweder einer der für die Erfindung erforderlichen UV-Filter fehlt oder die Zusammensetzungen in einer anderen Grundlage (insbesondere W/O-Emulsion) vorliegen. Darüber hinaus kennt der Stand der Technik "Neues aus Dermatologie, Kosmetik und Wundversorgung-Beiträge zum Stand der Technik, Nr. 18, Oktober 2007, die WPI Database Einträge XP-002563281, XP-002563280, die EP 1566172, das International Cosmetic Ingredient Dictionary and Handbook, 11th edition, volume 1, 2006, Seite 252 sowie "the Chemistry and Manufacture of Cosmetics", Volume II-Formulating, 2009, Seiten 73-99, doch konnten diese Schriften nicht den Weg zur vorliegenden Erfindung weisen.

Der erfindungsgemäß bevorzugte Süßholzextrakt stammt aus der Pflanze *Glycyrrhiza inflata.*

Erfindungsgemäß ist ferner vorteilhaft, wenn der Extrakt in Form eines wäßrigen Auszugs vorliegt, in welchem
- Licochalkon A
- Wasser
- gegebenenfalls ein oder mehrere Polyole
vorliegen.

Es ist erfindungsgemäß vorteilhaft, wenn die erfindungsgemäße kosmetische O/W-Emulsion, die erfindungsgemäße Verwendung oder das erfindungsgemäße Verfahren dadurch gekennzeichnet ist, dass die Zubereitung bzw. UV-Filterkombination als weiteren Bestandteil 2,4-Bis-{[4-(2-ethyl-hexyloxy)-2-hydroxy]-phenyl}-6-(4-methoxyphenyl)-1,3,5-triazin (INCI: Aniso Triazin) enthält.

Wenn im Rahmen der vorliegenden Beschreibung von erfindungsgemäßen Zubereitungen oder vorteilhaften Ausführungsformen etc. die Rede ist, bezieht sich dieser Umstand auch immer auf die erfindungsgemäße Verwendung und das erfindungsgemäße Herstellungsverfahren bzw. das erfindungsgemäße Verfahrensprodukt.

Erfindungsgemäß vorteilhafte Ausführungsformen der vorliegenden Erfindung dadurch gekennzeichnet, dass die Zubereitung mindestens 2,1 Gew.%, bezogen auf das Gesamtgewicht der Zubereitung, an 4-(tert.-Butyl)-4'-methoxydibenzoylmethan enthält.

Es ist generell erfindungsgemäß bevorzugt, die erfindungsgemäßen Bestandteile in den folgenden Konzentrationen einzusetzen:
a) 2-Ethylhexyl-2-cyano-3,3-diphenylacrylat (INCI: Octocrylene) in einer Konzentration von 0,1 bis 12 Gewichts- %, bezogen auf das Gesamtgewicht der Zubereitung.
b) 4-(tert.-Butyl)-4'-methoxydibenzoylmethan (INCI: Butylmethoxydibenzoylmethan) in einer Konzentration von 0,1 bis 8 Gewichts-%, bezogen auf das Gesamtgewicht der Zubereitung.
c) Süßholzextrakt in einer Konzentration von 0,001 bis 0,05 Gewichts-%, bezogen auf das Gesamtgewicht der Zubereitung.
d) Licochalcon A in einer Konzentration von 0,0001 bis 0,01 Gewichts-%, bezogen auf das Gesamtgewicht der Zubereitung.

2,4-Bis-{[4-(2-ethyl-hexyloxy)-2-hydroxy]-phenyl}-6-(4-methoxyphenyl)-1,3,5-triazin wird erfindungsgemäß vorteilhaft in einer Konzentration 0,1 von bis 5 Gewichts-%, bezogen auf das Gesamtgewicht der Zubereitung, eingesetzt.

Es ist erfindungsgemäß vorteilhaft, wenn als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat, Natrium Stearoyl Glutamat, Sucrose Polystearate + Hydrogenated Polyisobuten Polyglyceryl-3 Methylglucose Distearate Triceteareth-4 Phosphate eingesetzt wird.

Erfindungsgemäß vorteilhafte Ausführungsformen sind auch dadurch gekennzeichnet, dass die Zubereitung einen oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxyldisiloxanyl)propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltrümino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-(4'-Di-neopentylaminobenzalmalonat)-6-(4"-butylaminobenzoat)-s-triazin, Titandioxid; Zinkoxid, Trisbiphenyl-Triazin 4-Dicyanomethylen-2,6-dimethyl-1,4-dihydropyridin-N-(ethyloxysulfatestersalz) das mono-Natriumsalz, enthält.

Es ist erfindungsgemäß vorteilhaft, wenn die erfindungsgemäße Zubereitung frei ist von p-Methylbenzylidencampher.

Darüber hinaus kann die erfindungsgemäße Zubereitung vorteilhafter Weise ein oder mehrere weitere Verbindungen mit Alkoholfunktion enthalten, beispielsweise Glycerin, 2-Methyl-1,3-propandiol, Pentan-1,2-diol, Hexan-1,2-diol, Heptan-1,2-diol, Octan-1,2-diol, Nonan-1,2-diol, Decan-1,2-diol, Propanole, Propan- und Butandiole.

Es ist erfindungsgemäß vorteilhaft, wenn die erfindungsgemäße Zubereitung ein oder mehrere Antioxidantien enthält.

Es ist erfindungsgemäß bevorzugt, wenn die Zubereitung eine oder mehrere Verbindungen gewählt aus der Gruppe der Verbindungen Tocopherol, Tocopherolacetat, 2,6-Di-tert-butyl-4-methylphenol enthält.

Die erfindungsgemäße Zubereitung kann darüber hinaus ein oder mehrere weitere Wirkstoffe enthalten, beispielsweise alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Niacinamid, Vitamin A bzw. seine Derivate.

Es ist erfindungsgemäß vorteilhaft, wenn die Zubereitung ein oder mehrere Polymere enthält. Erfindungsgemäß bevorzugte Ausführungsformen sind dadurch gekennzeichnet, dass die Zubereitung ein oder mehrere Polymere gewählt aus der Gruppe der Verbindungen Acrylat/C10-C30 Alkylacrylat Crosspolymer, Acrylate, Carbomere, Tapiokastärke, Xanthangummi enthält.

Es ist vorteilhaft im Sinne der vorliegenden Erfindung, wenn die Ölphase der O/W-Emulsion ein oder mehrere Ölkomponenten gewählt aus der Gruppe der Verbindungen Butylenglycol Dicaprylat/Dicaprate, Myristylmyristat, Octyldodecanol, C12-15 Alkyl Benzoat Caprylic/Capric Triglyceride, Disopropyl Sebacate, Dicaprylyl Ether, Mineralöl, Silikonöl enthält.

Es ist erfindungsgemäß vorteilhaft, wenn die erfindungsgemäße Zubereitung ein oder mehrere Konservierungsmittel, insbesondere ein oder mehrere Parabene und/oder Phenoxyethanol enthält.

Darüber hinaus kann die erfindungsgemäße O/W-Emulsion weitere kosmetischen Wirk-, Hilfsund Zusatzstoffe enthalten, beispielsweise EDTA und Parfümstoffe wie Limonene, Linalool, Benzyl Benzoate, Hydroxyisohexyl 3-Cyclohexene Carboxaldehyde, Hexyl Cinnamal, Benzyl Salicylate, Eugenol, Butylphenyl Methylpropional, Alpha-Isomethyl lonone, Citronellol, Coumarin, Geraniol, Cinnamyl Alcohol, Citral

Ferner können in der Emulsion Sensorikadditive wie Distarch Phosphate Natrium Stärke Octenylsuccinate, Aluminium Stärke Octenylsuccinate, Acrylonitrile-methacrylonitrile-methylmethacrylate Copolymer + Isopentane + Magnesium Hydroxide, Tapioca Stärke, Silica, Nylon-6 Polyamide-5, Talkum mikron

Das erfindungsgemäße Herstellungsverfahren und das durch das Herstellungsverfahren hergestellte Produkt sind erfindungsgemäß durch die folgenden Details als erfindungsgemäß vorteilhaft gekennzeichnet:
So ist es erfindungsgemäß vorteilhaft, wenn der oder die Emulgatoren der Ölphase zugesetzt wurden bevor diese zur wässrigen Phase zugefügt wird.

Die heiße Wasserphase weist erfindungsgemäß vorteilhaft eine Temperatur von 50 bis 95 °C, bevorzugt eine Temperatur von 75 bis 90 °C und besonders bevorzugt eine Temperatur von etwa 85 °C auf. Der Begriff "etwa" soll dabei alleine die für derartige Verfahren typischen leichten Temperaturschwankungen kennzeichnen.

Die erhitzte Ölphase weist erfindungsgemäß vorteilhaft eine Temperatur von 50 bis 95 °C, bevorzugt eine Temperatur von 75 bis 90 °C und besonders bevorzugt eine Temperatur von etwa 85 °C auf. Der Begriff "etwa" soll dabei alleine die für derartige Verfahren typischen leichten Temperaturschwankungen kennzeichnen.

Als Homogenisatoren werden erfindungsgemäß vorteilhaft Mischer der Firma Becomix oder Krieger verwendet.

Die Homogenisierung erfolgt erfindungsgemäß vorteilhaft in einem Zeitraum von 2 bis 25 Minuten.

Die Rührgeschwindigkeit bei der Homogenisierung beträgt erfindungsgemäß vorteilhaft von 500 bis 2000 U/min., wobei eine Rührgeschwindigkeit von 1000 bis 1400 U/min. erfindungsgemäß bevorzugt, und eine Rührgeschwindigkeit von 1200 U/min (+-50 U/min.) erfindungsgemäß besonders bevorzugt ist.

Im Verfahrensschritt d) wird die Zubereitung erfindungsgemäß vorteilhaft auf eine Temperatur von 25 bis 40°C abgekühlt, wobei eine Temperatur von etwa 35°C bevorzugt ist. Der Begriff "etwa" soll dabei alleine die für derartige Verfahren typischen leichten Temperaturschwankungen kennzeichnen.

Die Parfümstoffe und Konservierungsmittel werden vor der Zugabe zur Emulsion bevorzugt miteinander vermischt.

Die Parfümstoffe und Konservierungsmittel werden vor der Zugabe zur Emulsion bevorzugt in Ethanol gelöst.

Die Zugabe der Parfümstoffe und Konservierungsmittel erfolgt erfindungsgemäß vorteilhaft bei einer Temperatur von unter 30 °C.

Vorteilhaft im Sinne der vorliegenden Erfindung können die Zubereitungen zur Pflege der Haut, dem kosmetischen Lichtschutz oder als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème. Tages- oder Nachtcrème usw. Es ist gegebenenfalls möglich und vorteilhaft, die erfindungsgemäßen Zusammensetzungen als Grundlage für pharmazeutische Formulierungen zu verwenden.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Vergleichsversuche

Mit den folgenden Vergleichsversuchen konnte der erfinderische Effekt gezeigt werden:
Es wurden die folgenden Rezepturen hergestellt und der Gehalt an Süßholzextrakt (enthaltend Licochalcon A) vor und nach einer 4 wöchigen Lagerung in einem Brutschrank bei 40 °C bestimmt.

Die Rezeptur Muster 1 enthält eine Zubereitung ohne die Stabilisatoren Butylmethoxydibenzoylmethan und Octocrylen. Die Rezeptur Muster 2 enthält zusätzlich die beiden Stabilisatoren.

| | **Muster 1** | **Muster 2** |
|---|---|---|
| **INCI-Name(n)** | **m[%]** | **m[%]** |
| Alcohol Denat. | 6,00 | 6,00 |
| Natrium Hydroxid | 0,60 | 0,60 |
| Cetearyl Alcohol + PEG-40 Castor Oil + Natrium Cetearyl Sulfat | 2,00 | 2,00 |
| Glyceryl Stearat SE | 0,80 | 0,80 |
| Trinatrium EDTA | 1,00 | 1,00 |
| Methylparaben | 0,30 | 0,30 |
| Phenoxyethanol | 0,20 | 0,20 |
| Bis-Ethylhexyloxyphenol Methoxyphenyl Triazin | 3,50 | 3,50 |
| **Butyl Methoxydibenzoylmethan** | | **4,50** |
| Diethylhexyl Butamido Triazon | 1,00 | 1,00 |
| Ethylhexyl Methoxycinnamat + BHT | 2,00 | 2,00 |
| **Octocrylene** | | **4,50** |
| Phenylbenzimidazol Sulfonsäure | 2,00 | 2,00 |
| Glycerin | 2,00 | 2,00 |
| Butylene Glycol Dicaprylate/Dicaprat | 7,00 | 7,00 |
| C12-15 Alkyl Benzoat | 11,00 | 11,00 |
| Myristyl Myristat | 2,00 | 2,00 |
| Octyldodecanol | 5,50 | 5,50 |
| Glycyrrhiza Inflata Root Extract (Licochalcon A) | 0,025 | 0,025 |
| Titandioxid + Trimethoxycaprylylsilan | 4,00 | 4,00 |
| Acrylates/C10-30 Alkyl Acrylat Crosspolymer | 0,05 | 0,05 |
| Cetyl Alcohol | 2,50 | 2,50 |
| Tapioca Starch | 1,00 | 1,00 |
| Xanthan Gum | 0,80 | 0,80 |
| Tocopheryl Acetat | 0,50 | 0,50 |
| Wasser | ad 100,000 | ad 100,000 |

### Versuchsdesign:

Bestimmung des Süßholzgehaltes (Licochalcon A) des Prüfmusters nach 4 Wochen Lagerstabilität bei 40°C Brutschrank Lagerung.

### Eingesetzte Methode:

Der Süßholzgehalt wurde mittels HPLC-DAD über eine externe Kalibrierreihe ermittelt. Zur Kalibrierung wurde das von der Auftraggeberin bereitgestellte Süßholz, NART: 96146-90000-00, Lot 17743901 eingesetzt. Die im Bericht angegebenen Gehalte beziehen sich auf die zur Kalibrierung eingesetzte Referenzsubstanz.

### Ergebnisse:

| **Probenbezeichnung** | **Gehalt in %** | |
|---|---|---|
| | **Ist** | **Soll** |
| Muster 1 | **< 0,005** | **0,025** |
| Muster 2 | **0,018** | **0,025** |

### Fazit:

Die Probe Muster 2 enthält mehr Süßholzextrakt (Licochalcon A) als die Probe Muster 1. Der Zusatz der Stabilisatoren Butylmethoxydibenzoylmethan und Octocrylen führt zu einer erhöhten Temperatur- und Lagerstabilität beim Süßholzextrakt (Licochalcon A).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

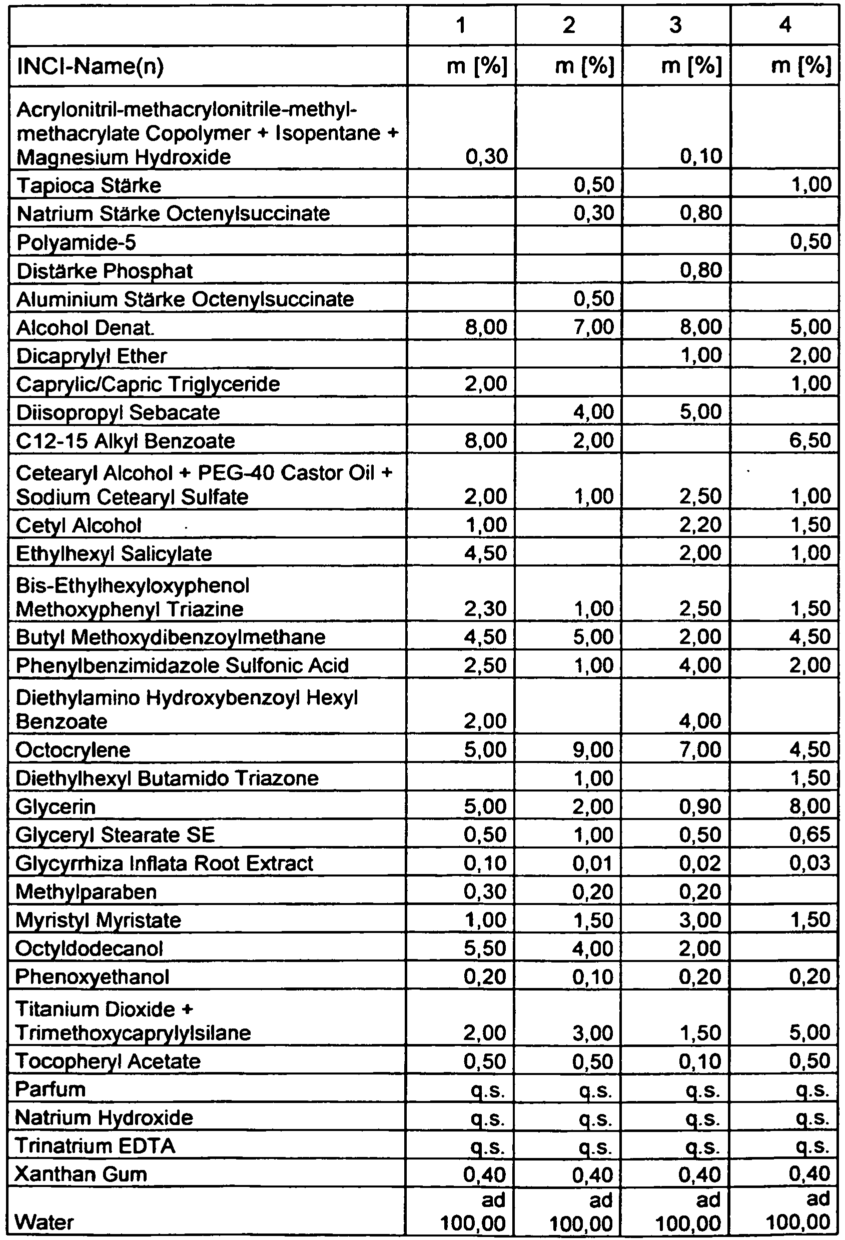

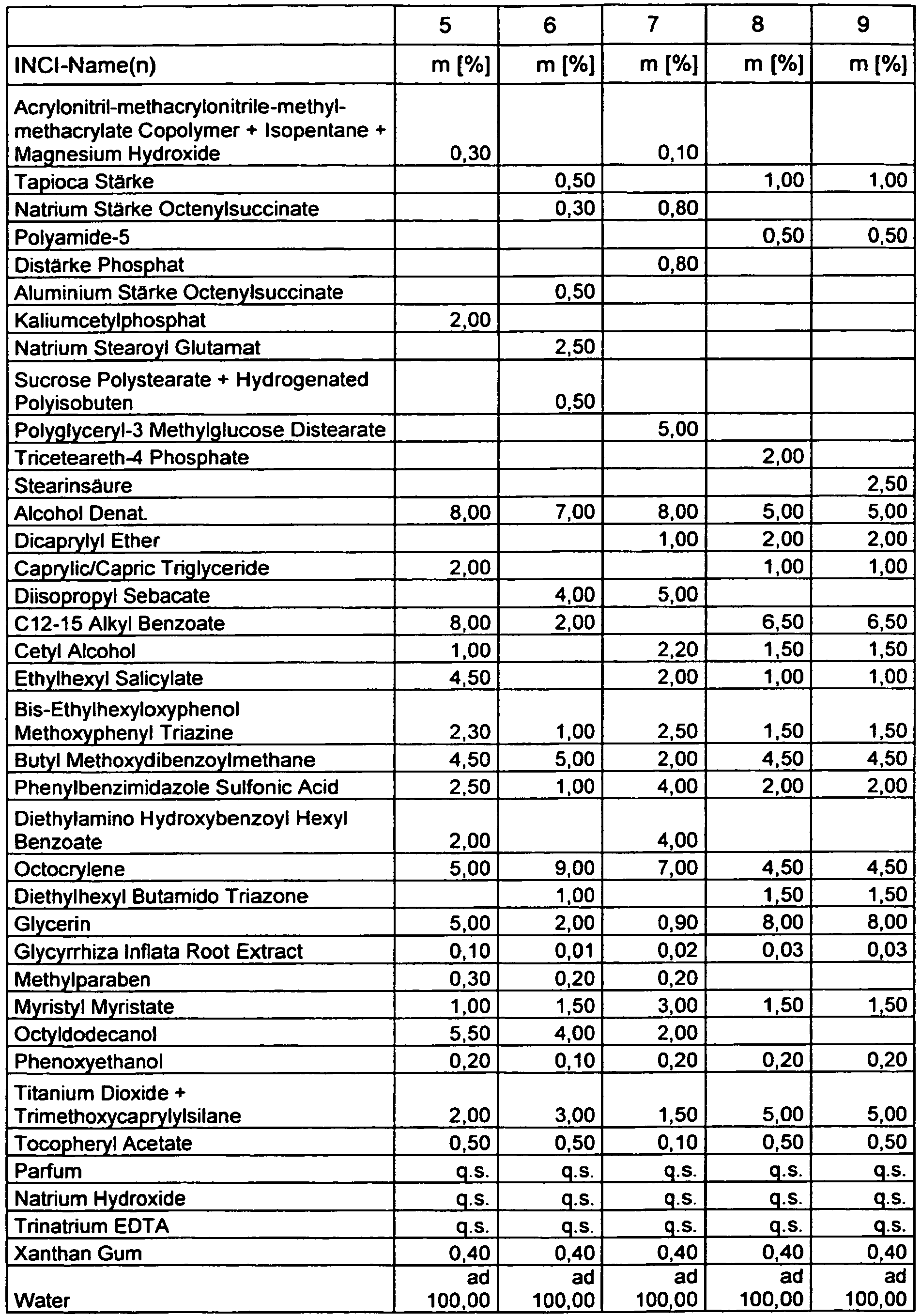

## Patentansprüche

1. Kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt und
d) Ethanol.

2. Kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Licochalcon A und
d) Ethanol.

3. Verwendung einer UV-Filterkombination aus
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan), zur Erhöhung der Lagerstabilität von Licochalkon A bzw. Süßholzextrakten in kosmetischen O/W-Emulsionen.

4. Verfahren zur Herstellung einer Licochalkon A und/oder Süßholzextrakt enthaltenden O/W-Emulsion, **dadurch gekennzeichnet, dass**
a) die heiße Wasserphase vorgelegt wird,
b) in die heiße Wasserphase die erhitzte Ölphase, enthaltend 2-Ethylhexyl-2-cyano-3,3-diphenylacrylat, 4-(tert.-Butyl)-4'-methoxydibenzoylmethan und ggf. weitere öllösliche UV-Filter wie 2,4-Bis-{[4-(2-ethyl-hexyloxy)-2-hydroxy]-phenyl}-6-(4-methoxyphenyl)-1,3,5-triazin, hinzugefügt wird,
c) anschließend beide Phasen zu einer O/W-Emulsion homogenisiert werden,
d) die Zubereitung abgekühlt wird,
e) der in Ethanol gelöste Süßholzextrakt bzw. das in Ethanol gelöste Licochalkon A in die Emulsion eingerührt wird und anschließend die Parfümstoffe und Konservierungsmittel zugesetzt werden.

5. Kosmetische O/W-Emulsion hergestellt nach einem Verfahren nach Anspruch 4.

6. Kosmetische O/W-Emulsion, Verwendung oder Verfahren nach einem der vorhergehenden Ansprüche, **dadurch gekennzeichnet, dass** die Zubereitung bzw. UV-Filterkombination als weiteren Bestandteil 2,4-Bis-{[4-(2-ethyl-hexyloxy)-2-hydroxy]-phenyl}-6-(4-methoxyphenyl)-1,3,5-triazin (INCI: Aniso Triazin) enthält.

7. Kosmetische O/W-Emulsion, Verwendung oder Verfahren nach einem der vorhergehenden Ansprüche, **dadurch gekennzeichnet, dass** die Zubereitung mindestens 2,1 Gew.%, bezogen auf das Gesamtgewicht der Zubereitung, an 4-(tert.-Butyl)-4'-methoxydibenzoylmethan enthält.

8. Kosmetische O/W-Emulsion, Verwendung oder Verfahren nach einem der vorhergehenden Ansprüche, **dadurch gekennzeichnet, dass** als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat eingesetzt wird.

9. Kosmetische O/W-Emulsion, Verwendung oder Verfahren nach einem der vorhergehenden Ansprüche, **dadurch gekennzeichnet, dass** die Zubereitung einen oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bomylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-(4'-Di-neopentylaminobenzalmalonat)-6-(4"-butylaminobenzoat)-s-triazin, Titandioxid; Zinkoxid, Trisbiphenyl-Triazin 4-Dicyanomethylen-2,6-dimethyl-1,4-dihydropyridin-N-(ethyloxysulfatestersalz) das mono-Natriumsalz, enthält.

10. Kosmetische O/W-Emulsion, Verwendung oder Verfahren nach einem der vorhergehenden Ansprüche, **dadurch gekennzeichnet, dass** die Zubereitung ein oder mehrere Antioxidantien enthält.

11. Kosmetische O/W-Emulsion, Verwendung oder Verfahren nach einem der vorhergehenden Ansprüche, **dadurch gekennzeichnet, dass** die Zubereitung eine oder mehrere Verbindungen gewählt aus der Gruppe der Verbindungen Tocopherol, Tocopherolacetat, 2,6-Di-*tert*-butyl-4-methylphenol enthält.

12. Kosmetische O/W-Emulsion, Verwendung oder Verfahren nach einem der vorhergehenden Ansprüche, **dadurch gekennzeichnet, dass** die Zubereitung ein oder mehrere Polymere enthält.

13. Kosmetische O/W-Emulsion, Verwendung oder Verfahren nach einem der vorhergehenden Ansprüche, **dadurch gekennzeichnet, dass** die Zubereitung ein oder mehrere Polymere gewählt aus der Gruppe der Verbindungen Acrylat/C10-C30 Alkylacrylat Crosspolymer, Acrylate, Tapiokastärke, Xanthangummi enthält.

14. Kosmetische O/W-Emulsion, Verwendung oder Verfahren nach einem der vorhergehenden Ansprüche, **dadurch gekennzeichnet, dass** die Zubereitung ein oder mehrere Ölkomponenten gewählt aus der Gruppe der Verbindungen Butylenglycol Dicaprylat/Dicaprate, Myristylmyristat, Octyldodecanol, Mineralöl, Silikonöl enthält.

## Claims

1. Cosmetic O/W emulsion comprising
a) 2-ethylhexyl 2-cyano-3,3-diphenylacrylate (INCI: octocrylene),
b) 4-(tert-butyl)-4'-methoxydibenzoylmethane (INCI: butyl methoxydibenzoyl methane),
c) licorice extract and
d) ethanol.

2. Cosmetic O/W emulsion comprising
a) 2-ethylhexyl 2-cyano-3,3-diphenylacrylate (INCI: octocrylene),
b) 4-(tert-butyl)-4'-methoxydibenzoylmethane (INCI: butyl methoxydi-benzoyl methane),
c) licochalcone A and
d) ethanol.

3. Use of a UV filter combination of
a) 2-ethylhexyl 2-cyano-3,3-diphenylacrylate (INCI: octocrylene),
b) 4-(tert-butyl)-4'-methoxydibenzoylmethane (INCI: butyl methoxydibenzoyl methane),
to increase the storage stability of licochalcone A or licorice extracts in cosmetic O/W emulsions.

4. Process for the preparation of an O/W emulsion comprising licochalcone A and/or licorice extract, **characterized in that**
a) the hot aqueous phase is initially introduced into the preparation vessel,
b) the heated oily phase containing 2-ethylhexyl 2-cyano-3,3-diphenylacrylate, 4-(tert-butyl)-4'-methoxydibenzoylmethane and optionally further oil-soluble UV filters, such as 2,4-bis-([4-(2-ethyl-hexyloxy)-2-hydroxy]-phonyll-6-(4-methoxyphenyl)-1,3,5-triazine, is added to the hot aqueous phase,
c) the two phases are subsequently homogenized to give an O/W emulsion,
d) the formulation is cooled,
e) the licorice extract dissolved in ethanol or the licochalcone A dissolved in ethanol is stirred into the emulsion, and the perfume substances and preservatives are subsequently added.

5. Cosmetic O/W emulsion prepared by a process according to Claim 4.

6. Cosmetic O/W emulsion, use or process according to one of the preceding claims, **characterized in that** the formulation or UV filter combination contains as a further constituent 2,4-bis-{[4-(2-ethyl-hexyloxy)-2-hydroxy]-phenyl}-6-(4-methoxyphenyl)-1,3,5-triazine (INCI. aniso triazine).

7. Cosmetic O/W emulsion, use or process according to one of the preceding claims, **characterized in that** the formulation contains at least 2.1 wt.%, based on the total weight of the formulation, of 4-(tert-butyl)-4'-methoxydibenzoylmethane.

8. Cosmetic O/W emulsion, use or process according to one of the preceding claims, **characterized in that** glyceryl stearate citrate, glyceryl stearate SE, stearic acid, polyglyceryl-3 methylglucose distearate, PEG-40 stearate, PEG-100 stearate, potassium cetyl phosphate and/or a mixture of cetearyl alcohol + PEG-40 hydrogenated castor oil + sodium cetearyl sulfate + glyceryl stearate is employed as an O/W emulsifier.

9. Cosmetic O/W emulsion, use or process according to one of the preceding claims, **characterized in that** the formulation contains one or more UV filters chosen from the group of the compounds phenylene-1,4-bis-(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)-benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylene-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetromethyl-1-[(trimethylsilyl)oxyldisiloxanyl]propyl]-phenol, 3-(4-methylbenzylidene)camphor; 3-benzylidenecamphor; ethylhexyl salicylate; terephthalidenedicamphorsulfonic acid; 4-(dimethylamino)-benzoic acid (2-ethylhexyl) ester; 4-(dimethylamino)benzoic acid amyl ester; 4-methoxybenzalmalonic acid di(2-ethylhexyl) ester; 4-methoxycinnamic acid (2-ethylhexyl) ester; 4-methoxycinnamic acid isoamyl ester; 2-hydroxy-4-methoxybenzophenone, 2-hydroxy-4-methoxy-4'-methylbenzophenone; 2,2'-dihydroxy-4-methoxybenzophenone; 2-(4'-diethylamino-2'-hydoxybenzoyl)-benzoic acid hexyl ester, homomenthyl salicylate; 2-ethylhexyl 2-hydroxybenzoate; dimethicodiethylbenzal malonate; 3-(4-(2,2-bis ethoxycarbonylvinyl)-phenoxy)propenyl)-methoxysiloxane / dimethylsiloxane copolymer; dioctylbutylamidotriazone (INCI: diethylhexyl-butamido triazone); 2,4-bis-[5-1 (dimethylpropyl)benzoxazol-2-yl-(4-phenyl)-imino]-6-(2-ethylhexyl)-imino-1,3,6-triazine with the (CAS no. 288254-16-0); 4,4',4"-(1,3,5-triazine-2,4,6-triyltriimino)-tris-benzoic acid tris(2-ethylhexyl ester) (also: 2,4,6-tris-[anilino-(p-carbo-2'-ethyl-1'-hexyloxy)]-1,3,5-triazine (INCI: ethylhexyl triazone); 2,4-bis-(4'-di-neopentylaminobenzalmalonate)-6-(4"-butylaminobenzoate)-s-triazine, titanium dioxide; zinc oxide, trisbiphenyl-triazine 4-dicyanomethylene-2,6-dimethyl-1,4-dihydropyridine N-(ethyloxysulfate ester salt) mono-sodium salt.

10. Cosmetic O/W emulsion, use or process according to one of the preceding claims, **characterized in that** the formulation contains one or more antioxidants.

11. Cosmetic O/W emulsion, use or process according to one of the preceding claims, **characterized in that** the formulation contains one or more compounds chosen from the group of the compounds tocopherol, tocopherol acetate, 2,6-di-*tert*-butyl-4-methylphenol.

12. Cosmetic O/W emulsion, use or process according to one of the preceding claims, **characterized in that** the formulation contains one or more polymers.

13. Cosmetic O/W emulsion, use or process according to one of the preceding claims, **characterized in that** the formulation contains one or more polymers chosen from the group of the compounds acrylate/C10-C30 alkyl acrylate cross polymer, acrylates, tapioca starch, xanthan gum.

14. Cosmetic O/W emulsion, use or process according to one of the preceding claims, **characterized in that** the formulation contains one or more oil components chosen from the group of the compounds butylene glycol dicaprylate/dicaprate, myristyl myristate, octyldodecanol, mineral oil, silicone oil.

## Revendications

1. Émulsion H/E cosmétique contenant
a) du 2-cyano-3,3-diphénylacrylate de 2-éthylhexyle (INCI: octocrylene),
b) du 4-(tert.-butyl)-4'-méthoxydibenzoylméthane (INCI: butylmethoxydibenzoylmethan),
c) de l'extrait de réglisse et
d) de l'éthanol.

2. Émulsion H/E cosmétique contenant
a) du 2-cyano-3,3-diphénylacrylate de 2-éthylhexyle (INCI: octocrylene),
b) du 4-(tert.-butyl)-4'-méthoxydibenzoylméthane (INCI: butylmethoxydibenzoylmethan),
c) de la licochalcone A et
d) de l'éthanol

3. Utilisation d'une association de filtres UV à base de
a) 2-cyano-3,3-diphénylacrylate de 2-éthylhexyle (INGI: octocrylene),
b) 4-(tert.-butyl)-4'-méthoxydibenzoylméthane (INCI: butylmethoxydibenzoylmethan),
pour augmenter la stabilité au stockage de licochalcone A ou d'extraits de réglisse dans des émulsions H/E cosmétiques.

4. Procédé pour la préparation d'une émulsion H/E contenant de la licochalcone A et/ou de l'extrait de réglisse, **caractérisé en ce que**
a) on dispose au préalable la phase aqueuse chaude,
b) on introduit dans la phase aqueuse chaude la phase huileuse chauffée, contenant du 2-cyano-3,3-diphénylacrylate de 2-éthylhexyle, du 4-(tert.-butyl)-4'-méthoxydibenzoylméthane et éventuellement d'autres filtres UV liposolubles tels que la 2,4-bis-{[4-(2-éthyl-hexyloxy)-2-hydroxy]-phényl}-6-(4-méthoxyphényl)-1,3,5-triazine,
c) ensuite on homogénéise les deux phases pour obtenir une émulsion H/E,
d) on refroidit la préparation,
e) on délaie dans l'émulsion l'extrait de réglisse dissous dans de l'éthanol ou la licochalcone A dissoute dans de l'éthanol et ensuite on ajoute les parfums et conservateurs.

5. Émulsion H/E cosmétique préparée conformément à un procédé selon la revendication 4.

6. Émulsion H/E cosmétique, utilisation ou procédé selon l'une quelconque des revendications précédentes, **caractérisés en ce que** la préparation ou l'association de filtres UV contient comme autre composant de la 2,4-bis-{[4-(2-éthyl-hexyloxy)-2-hydroxy]-phényl}-6-(4-méthoxyphényl)-1,3,5-triazine (INCI: aniso triazin).

7. Émulsion H/E cosmétique, utilisation ou procédé selon l'une quelconque des revendications précédentes, **caractérisés en ce que** la préparation contient au moins 2,1 % en poids, par rapport au poids total de la préparation, de 4-(tert.-butyl)-4'-méthoxydibenzoylméthane.

8. Émulsion H/E cosmétique, utilisation ou procédé selon l'une quelconque des revendications précédentes, **caractérisés en ce qu'**on utilise comme émulsifiant H/E le citrate-stéarate de glycéryle, le stéarate de glycéryle 5E, l'acide stéarique, le distéarate de polyglycéryl-3-méthylglucose, le stéarate de PEG-40, le stéarate de PEG-100, le cétylphosphate de potassium et/ou un mélange d'alcool cétéarylique + PEG-40 huile de ricin hydrogénée + cétéarylsulfate de sodium + stéarate de glycéryle.

9. Émulsion H/E cosmétique, utilisation ou procédé selon l'une quelconque des revendications précédentes, **caractérisés en ce que** la préparation contient un ou plusieurs filtres UV choisis dans le groupe des composés sels d'acide phénylène-1,4-bis-(2-benzimidazyl)-3,3'-5,5'-tétrasulfonique ; sels d'acide 2-phénylbenzimidazole-5-sulfonique ; 1,4-di(2-oxo-10-sulfo-3-bornylidèneméthyl)-benzène et ses sels ; sels d'acide 4-(2-oxo-3-bornylidèneméthyl)benzènesulfonique ; sels d'acide 2-méthyl-5-(2-oxo-3-bornylidèneméthyl)sulfonique ; 2,2'-méthylène-bis(6-(2H-benzotriazol-2-yl)-4-(1,1,3,3-tétraméthylbutyl)-phénol); 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidénedicamphosulfonique ; 4-(diméthyiamino)benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; 2-(4'-diéthylamino-2'-hydroxybenzoyl)-benzoate d'hexyle ; salicylate d'homomenthyle ; 2-hydroxybenzoate de 2-éthylhexyle; benzalmalonate de diméthicodiéthyle ; copolymère 3-(4-(2,2-biséthoxycarbonylvinyl)-phénoxy)propényl)-méthoxysiloxane / diméthylsiloxane ; dioctylbutylamidotriazone (INCI : diethylhexyl-butamidotriazone) ; 2,4-bis-[5-1 (diméthylpropyl)benzoxazol-2-yl-(4-phényl)-imino]-6-(2-éthylhexyl)-imino-1,3,5-triazine portant le (n° CAS 288254-16-0) ; 4,4',4"-(1,3,5-triazine-2,4,6-triyltriimino)-tris-benzoate de tris(2-éthylhexyle) (également : 2,4,6-tris[anilino-(p-carbo-2'-éthyl-1'-hexyloxy)]-1,3,5-triazine (INCI : ethylhexyl triazone) ; 2,4-bis-(4'-di-néopentylaminobenzalmalonate)-6-(4"-butylaminobenzoate)-s-triazine, dioxyde de titane, oxyde de zinc, trisbiphényltriazine, sel monosodique de 4-dicyanométhylène-2,6-diéthyl-1,4-dihydropyridine-N-(sel éthyloxysulfate-ester).

10. Émulsion H/E cosmétique, utilisation ou procédé selon l'une quelconque des revendications précédentes, **caractérisés en ce que** la préparation contient un ou plusieurs oxydants.

11. Émulsion H/E cosmétique, utilisation ou procédé selon l'une quelconque des revendications précédentes, **caractérisés en ce que** la préparation contient un ou plusieurs composés choisis dans le groupe des composés tocophérol, acétate de tocophérol, 2,6-di-tert-butyl-4-méthylphénol.

12. Émulsion H/E cosmétique, utilisation ou procédé selon l'une quelconque des revendications précédentes, **caractérisés en ce que** la préparation contient un ou plusieurs polymères.

13. Émulsion H/E cosmétique, utilisation ou procédé selon l'une quelconque des revendications précédentes, **caractérisés en ce que** la préparation contient un ou plusieurs polymères choisis dans le groupe des composés acrylat/C10-C30 alkylacrylat crosspolymer, acrylates, amidon de tapioca, gomme xanthane.

14. Émulsion H/E cosmétique, utilisation ou procédé selon l'une quelconque des revendications précédentes, **caractérisés en ce que** la préparation contient un ou plusieurs composants huileux, choisis dans le groupe des composés dicaprylate/dicaprate de butylèneglycol, myristate de myristyle, octyldodécanol, huile minérale, huile de silicone.
